# EUROPEAN PATENT SPECIFICATION

(11) **EP 2 396 549 B1**
(45) Date of publication and mention of the grant of the patent: **23.10.2019**
(21) Application number: 10741820.4
(22) Date of filing: 12.02.2010
(51) Int. Cl.: F04B 43/12, F04B 43/08, A61M 1/10

(54) **MODULAR FLUID PUMP WITH CARTRIDGE**
MODULARE FLÜSSIGKEITSPUMPE MIT PATRONE
POMPE MODULAIRE DOTÉE D'UNE CARTOUCHE

(30) Priority: 12.02.2009 US 152182 P
(43) Date of publication of application: 21.12.2011
(73) Proprietor: MC3, Inc., Ann Arbor, MI 48103 (US)
(72) Inventor: MAZUR, Daniel, E., Ann Arbor MI 48103 (US)
(74) Representative: Mathys & Squire
(86) International application number: PCT/US2010/024129
(87) International publication number: WO 2010/093946

(56) References cited:
- US-A- 3 756 752
- US-A- 4 515 535
- US-A- 5 281 112
- US-A- 5 658 136
- US-A1- 2006 233 648

## Description

### CROSS-REFERENCE TO RELATED APPLICATIONS

This application claims the benefit of US Provisional Application number 61/152,182, filed 12 FEB 2009.

### TECHNICAL FIELD

This invention relates generally to the fluid pump field, and more specifically to an improved modular fluid pump in the fluid pump field.

Reference is directed to US 5,281,112 which discloses a peristaltic pump having controlled suction. In one embodiment, the pump includes a tubing which is collapsed when the internal pressure of the tubing is equal to or less than ambient. In this state, the pump is unable to generate negative pressures. An air-tight housing may be provided over the tubing and connected to a vacuum source. Upon a vacuum being applied, the tubing is capable of controllably producing negative pressures, allowing the pump to be operated as a suction pump.

Reference is further directed to US 5,658,136 which discloses a centrifugal blood pump which has a pump rotor arranged inside a housing which is closed with the exception of a blood outlet and a blood inlet. The rotatable pump rotor inside the housing is driven by an external drive motor via a magnetic coupling. The magnetic coupling is provided by thin exterior permanent magnets circumferentially distributed around the rotor on a coupling part connected to the external drive motor and correspondingly thin circumferentially distributed permanently magnetized ferromagnetic regions of the pump rotor which are coupled with the permanent magnets to form a magnetic field bridge and thus to avoid axially directed tilting or tipping moments. The pump rotor can be additionally stabilized against translational motion in the radial direction by a mechanical bearing device for the rotor which is provided inside an interior space through which blood flows in the pump housing.

Reference is further directed to US 3,756,752 which discloses a peristaltic pump including a readily replaceable tube with nipples affixed to the tube ends and which fit and are anchored by sockets in the pump casing, and a rotor disk provided with a tube guide for guiding a replacement tube into operative position in the casing.

### BACKGROUND

In the medical field, blood pumps are commonly used to provide extracorporeal life support to patients lacking sufficient cardiac function. For example, patients with cardiac failure may require cardiac support long enough to sustain the patient until recovery, heart transplantation, or implantation of a permanent artificial cardiac device. As another example, a patient undergoing cardiac bypass surgery require external means of pumping blood throughout their circulatory system. One kind of pump often used in such extracorporeal life support systems is a roller pump. In a typical roller pump, rollers press against a piece of tubing that is wrapped around the rollers and backed by a rigid raceway to advance fluid through the tubing. Roller pumps typically draw blood from a patient or a venous reservoir of blood supplied by the patient. However, roller pumps typically provide no inherent means of preventing draining of the venous reservoir, and if left unattended, will drain the venous reservoir and continue to pump air to the patient, which may be dangerous for the patient. Furthermore, the tubing in a roller pump has a limited lifetime due to repeated stress during operation, and must periodically be replaced. However, optimal operation of a roller pump typically requires an experienced and skilled operator to carefully set settings on the pump, such as occlusion settings (effectively the maximum amount of compression exerted on the tubing) or tubing tension (wrapping the tubing around the rollers with the proper amount of tension) to optimize the performance of the pump and reduce harmful effects to the patient. Thus, there is a need in the fluid pump field to create an improved modular fluid pump. This invention provides such an improved modular fluid pump.

### BRIEF DESCRIPTION OF THE FIGURES

FIGURES 1A-1C are a perspective view, plan view, and cross sectional view, respectively, of the fluid pump of a preferred embodiment;
FIGURE 2 is a non-contact variation of the drive coupling means in the fluid pump of a preferred embodiment;
FIGURE 3 is a contact variation of the drive coupling means in the fluid pump of a preferred embodiment;
FIGURES 4A and 4B are a side view and a cross-sectional view, respectively, of the fluid conduit operating in the filled mode, in the fluid pump of a preferred embodiment;
FIGURES 5A and 5B are a side view and a cross-sectional view respectively, of the fluid conduit operating in the collapsed mode, in the fluid pump of a preferred embodiment; and
FIGURE 6 is a schematic of the method of supplying a plurality of housing cartridges of a preferred embodiment.

### DESCRIPTION OF THE PREFERRED EMBODIMENTS

The following description of preferred embodiments of the invention is not intended to limit the invention to these preferred embodiments, but rather to enable any person skilled in the art to make and use this invention.

### 1. Modular fluid pump system

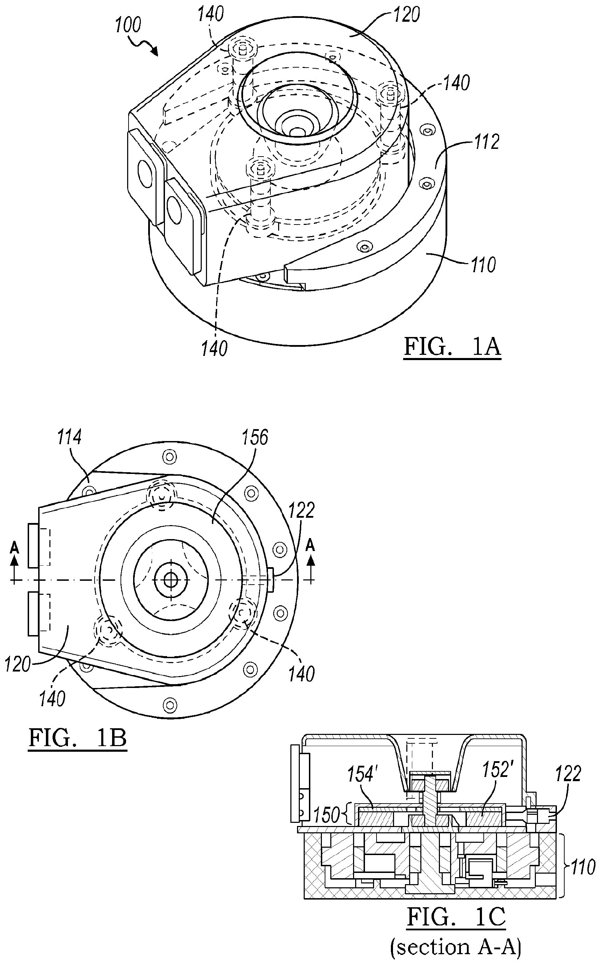

As shown in FIGURE 1, the modular fluid pump 100 of the preferred embodiment includes: a pump motor 110 that provides a motor force; a housing cartridge 120 removably coupled to the pump motor 110, in which the housing cartridge 120 is interchangeable with a replacement housing cartridge; a frame 130 enclosed in the housing cartridge 120 and having a plurality of rollers 140 arranged on the frame 130; a drive coupling means 150 between the pump motor 110 and the frame 130, wherein the drive coupling means 150 transmits the motor force to the frame 130 to induce rotation of the frame 130; and a fluid conduit 160, wrapped under tension around at least one of the plurality of rollers 140, that facilitates fluid flow from an inlet region 162 of the fluid conduit 160 to an outlet region 166 of the fluid conduit 160. The rotation of the frame 130 preferably causes the plurality of rollers 140 to compress the fluid conduit 160 at subsequent intervals, thereby driving fluid to the outlet region 166 of the fluid conduit with a peristaltic movement. The modular fluid pump 100 preferably is used to pump blood and is part of an extracorporeal life support system. The fluid pump 100 preferably interfaces with the circulatory system of the patient, receiving blood of the patient and pumping the blood to return to the patient. The fluid pump 100 preferably provides an outlet fluid pump pressure no more than a predetermined safe level and maintains safe levels of inlet region pressure to avoid harmful effects to the patient such as cavitation (formation of vapor bubbles in the fluid) and hemolysis (the lysis, or breaking open, of red blood cells). The fluid pump 100 is preferably a Starling pump that provides a flow output dependent on the flow input, and is preferably responsive to variations in hemodynamic changes of the patient, acting as an automatically adaptive reservoir. The housing cartridge 120 of the fluid pump is preferably interchangeable with a replacement housing cartridge to provide a simple, easy way to replace the pump head portion (e.g., the fluid conduit and rollers) of the fluid pump 100 due to reasons such as maintenance or wear. This interchangeability of housing cartridges increases the reliability and consistency of the fluid pump between uses of the fluid pump, reduces risk of user error that could be harmful to the patient, and simplifies use and maintenance of the fluid pump. The fluid pump may be used in any suitable application of a cardiac life support system, such supporting a patient during a cardiac bypass procedure, or supporting a patient with cardiac failure long enough to sustain the patient until recovery, heart transplantation, or implantation of a permanent artificial cardiac device. Furthermore, the fluid pump may interface with an oxygenator or other gas exchange device to provide a patient with cardiorespiratory support. The fluid pump may, however, be used to pump any suitable fluid.

The pump motor 110 of the preferred embodiment functions to provide a motor force actuation for the plurality of rollers 140 on the frame 130. The pump motor 110 is preferably a direct drive, brushless DC motor with high torque at low RPM, which increases reliability and efficiency that may be lost in a speed reduction gearhead and reduces audible noise. A brushless DC motor also typically has a relatively low emission of electromagnetic radiation, which reduces the likelihood of the fluid pump interfering with other nearby medical and electronic devices. However, the pump motor may be a brushed DC motor, a stepper motor, a single or polyphase AC motor, or any suitable actuator, and may be coupled to a transmission such as a geartrain and/or pulley system to increase the torque output and/or direct the output in a particular orientation. The exact specifications of the pump motor 110 is preferably selected based on the desired application of the fluid pump; for example, in a fluid pump intended for pediatric patients, the pump motor 110 preferably has a smaller physical size and provides a lower torque output to satisfy a lower required torque. In some variations, the pump motor preferably is coupled to a motor housing 112. As shown in FIGURE 1B, the motor housing 112 preferably couples to a face of the pump motor 110 that receives the housing cartridge 120, but the motor housing may alternatively enclose the pump motor. The motor housing 112 preferably includes a mating surface 114 that mates with the housing cartridge 120. As best shown in FIGURE 1A, the motor housing 112 preferably includes a circular lip forming a recess that receives the housing cartridge 120 in a sliding manner, but may additionally and/or alternatively include any suitable shaped lip, recess, or other mating surface into which the housing cartridge 120 slides or otherwise engages with. The motor housing 112 is preferably biased, such as with a taper or a keyway, such that the housing cartridge 120 is constrained to couple to the motor housing or pump motor in a particular orientation, which helps ensure that the housing cartridge is placed appropriately for correct pump operation. The housing cartridge 120 may additionally and/or alternatively include tabs, notches, latches, magnetic snaps, or other mechanisms that help lock the housing cartridge 120 into place on the mating surface 114 of the motor housing 112, thereby securely coupling the housing cartridge 120 to the pump motor 110. However, the fluid pump may alternatively lack a motor housing 112, such that the housing cartridge 120 removably couples solely to the pump motor 110.

The housing cartridge 120 of the preferred embodiment functions to enclose the pump head components and to removably couple with the pump motor 110 such that the housing cartridge 120 is interchangeable with a replacement housing cartridge. The housing cartridge 120 is preferably substantially airtight sealed and includes a port 122 that facilitates adjustment of the pressure within the housing cartridge. The pressure within the housing may be a fixed or variable subatmospheric or superatmospheric pressure. In one variation, the pressure control port 122 is a vacuum port that couples to a vacuum pump 180 and facilitates formation of a vacuum (i.e., negative pressure) within the housing cartridge. In another variation, the pressure control port 122 couples to a pump and facilitates a positive pressure within the housing cartridge. The housing cartridge 120 preferably further includes inlet and outlet conduit ports with mounted fittings, through which the fluid conduit 160 enters and exits, respectively, the housing cartridge. The housing cartridge 120 is preferably of unitary construction, which increases the robustness of the airtight seal. However, the housing cartridge may alternatively be composed of multiple separate pieces joined together by fasteners, epoxy, a welding process, and/or any suitable joining process. In this variation, the joints between the separate housing cartridge pieces are preferably sealed with a welding process, gasket, or sealant to make the housing cartridge 120 airtight sealed. Similar sealing processes are preferably used to seal the conduit ports, and any other openings in the housing cartridge. The sealing limits exposure of a user to the fluid within the tubing and the housing cartridge, by not requiring contact between the user and the fluid during removal of the housing cartridge from the fluid pump. The sealing also limits fluid that is potentially lost in a failure such as a tubing leak. As shown in FIGURES 1A and 1B, the housing cartridge 120 preferably includes a generally round portion, and has a width that is greater than its depth. The round portion preferably provides clearance for the rotation of the frame 130. The depth of the housing cartridge 120 is preferably deep enough to accommodate the length of the plurality of rollers 140 on the frame 130, but not too deep, to avoid excess space in which the fluid conduit 160 may wander along the length of and/or slip off the plurality of rollers 140 during pump operation. An overall smaller housing cartridge volume will also decrease noise and vibration produced by the vacuum pump 180 and the fluid pump. The housing cartridge 120 is preferably made of plastic, and more preferably a transparent plastic such as polycarbonate that allows an operator to monitor the operation of the pump head components inside the housing cartridge 120 and increase the likelihood of a quick response to a malfunction, such as a fluid conduit leak. However, the housing cartridge may be made of any suitable material with low magnetic permeability (low degree of magnetization in a magnetic field), or any suitable material. The housing cartridge 120 is preferably injected molded, but may alternatively be machined on a mill, casted, or created through any suitable manufacturing process. In one variation, the housing cartridge 120 is removable for disposal and/or recycling. In another variation, the housing cartridge 120 is removable for refurbishing and reuse, during which consumable parts such as the fluid conduit 160 are replaced and/or repaired, and the housing cartridge 120 and its pump head contents are preferably reassembled to an appropriate standard for use as a replacement housing cartridge.

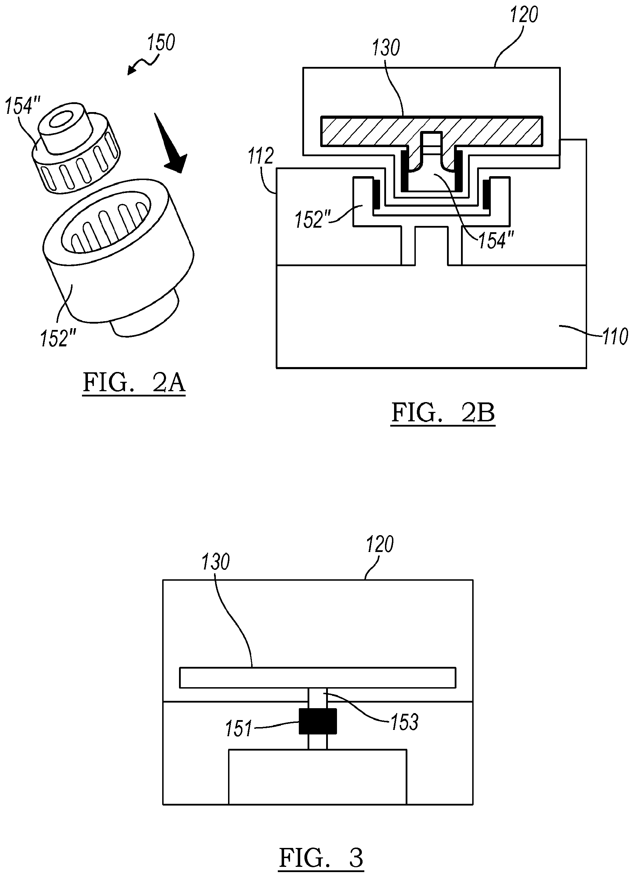

The frame 130 of the preferred embodiment functions to support a plurality of rollers 140 and provide the movement of a plurality of rollers 140 along a substantial length of the fluid conduit 160. The frame 130 is preferably enclosed in the housing cartridge 120 and is operatively coupled to the motor force of the pump motor 110 through the non-contact coupling means 150, such that the motor force induces rotation of the frame 130. The frame 130 preferably includes a plurality of rollers 140. As shown in FIGURES 1 and 3, the rollers 140 are preferably distributed in a plane around the frame 130, preferably extend generally parallel to the axis of rotation of the frame, and preferably are equally spaced apart to form the vertices of a regular polygon. For example, the frame 130 may include three rollers arranged at the vertices of an equilateral triangle. As the frame 130 rotates, the plurality of rollers 140 preferably move in a generally circular path. However, the number and arrangement of rollers 140 on the frame 130 may depend on the specific application. For example, the rollers 140 may be arranged in an irregular distribution such that the rollers travel in a noncircular path. Each of the rollers 140 is preferably mounted for free rotation about its own axis, such that as the rollers 140 move along the fluid conduit 160 and do not produce a significant amount of friction on the fluid conduit. The rollers may have a low friction coating or lubricant to further reduce the amount of friction the fluid conduit 160, which may reduce degradation of the fluid conduit and increase the useful life of the fluid conduit.

The drive coupling means 150 of the preferred embodiment functions to transmit the motor force to the frame 130, thereby inducing rotation of the frame during operation of the fluid pump. The drive coupling means 150 is preferably one of several variations. In a first variation, the drive coupling means is a non-contact coupling means. The non-contact coupling means preferably includes a magnetic coupler with a first mating portion 152 and a second mating portion 154 magnetically attracted to the first mating portion 152. The first mating portion 152 of the magnetic coupler is preferably connected to the rotor of the pump motor 110, and the second mating portion 154 of the magnetic coupler is preferably connected to the frame 130. Because the first and second mating portions of the magnetic coupler are magnetically attracted, movement of the pump motor rotor is tracked by the frame, such that rotation and motor force of the pump motor is transmitted into rotation of the frame 130. Since the magnetic field of the magnetic coupling preferably passes through the material of the housing cartridge, the magnetic coupler allows the housing cartridge 120 to be self-contained without the need for additional mechanical drive coupling between the pump motor 110 and the frame 130 that would complicate the coupling between the pump motor 110 and housing cartridge 120. This simplifies the removable coupling between the housing cartridge and pump motor, in that the housing cartridge 120 is preferably interchangeable with a quick, drop-in replacement. The magnetic coupler preferably includes permanent magnets for reliable drive coupling. However, the magnetic coupler may alternatively include electromagnets that can be controlled to selectively provide the magnetic attraction forming the drive coupling, which may have certain advantages in some applications. For example, since removing electric current to an electromagnetic drive coupling removes the magnetic field, such selective decoupling of the frame 130 and pump motor 110 may be useful in quick shut-off situations, or to facilitate quick removal of the housing cartridge 120 for replacement of the housing cartridge.

The magnetic coupler is preferably one of several variations. In a first variation, as shown in FIGURE 1, the magnetic coupler is a magnetic disc coupler 156, in which the first mating portion 152' is a first magnetic disc and the second mating portion 154' is a second magnetic disc that is magnetically attracted to the first disc. In a second variation, as shown in FIGURE 2, the magnetic coupler is a magnetic coaxial coupling 158, in which the first mating portion 152" and second mating portion 154" are coaxial, magnetically attracted nested hubs. In a third variation, the magnetic coupler includes magnetic shielding that modulates and directs the magnetic field to induce rotation of the frame 130. In this variation, magnetic shielding is preferably made of a material that has high magnetic permeability and high magnetic saturation to enable the magnetic shielding to easily reroute magnetic field lines. The magnetic shield preferably includes at least one opening or other geometric relief of the rerouting of magnetic field lines. The magnetic shielding is preferably coupled to the pump motor 110 and positioned between the first and second mating portions of the magnetic coupler. As the pump motor rotor rotates, the magnetic shielding rotates, and the opening of the magnetic shielding through which magnetic attraction is not blocked rotates, thereby allowing the frame 130 of the fluid pump to track the rotation of the pump motor 110. In any of these variations, the magnetic coupler may alternatively include first and second mating portions of identical poles, such that the first and second mating portions are magnetically repulsive and the movement of the pump motor rotor repels, or "pushes", the frame 130 into rotation. However, the magnetic coupler may be a combination of these variations and/or of additional variations that incorporate magnetic force as a drive coupling means 150. Furthermore, the non-contact drive coupling means may be any suitable indirect coupling means that transmits the motor force into a rotation of the frame 130.

In a second variation, as shown in FIGURE 3, the drive coupling means 150 between the frame 130 and the pump motor 110 includes a direct contact coupling means between the frame 130 and the pump motor, such as a drive shaft 153 or flexible drive coupling. The drive coupling means 150 may include keyways or other features that enhance the robustness of the drive coupling means between the frame 130 and the pump motor 110.

In a third variation, the drive coupling means 150 is between the frame 130 and a manual actuator. In this variation, the manual actuator is a hand crank, a hand pump, or any suitable hand operable actuator that may be coupled to the frame to induce rotation of the frame.

Other embodiments may incorporate a combination of the variations of non-contact and contact coupling means. In any of these variations, as shown in FIGURE 3, the drive coupling means 150 may include a torque limiting feature or coupling device 151 that provides a fail-safe feature of the fluid pump. The drive coupling means 150 of the first variation inherently includes a torque limiting feature, since the magnetic forces can only couple the two mating portion with a limited force. The torque limiting coupling device 151 may include a torque-limiting clutch that slips and/or separates the coupling at an overload above a certain predetermined level of torque to avoid driving the fluid pump at an excessive level of torque. For example, the torque-limiting clutch may be an overload clutch (which disengages and remains disengaged until re-engaged manually or with other devices), a ratchetting clutch (which disengages and re-engages within any of a number of segments around the circumference of the clutch), or a synchronized clutch (which disengages and re-engages at the same point around the circumference of the clutch).

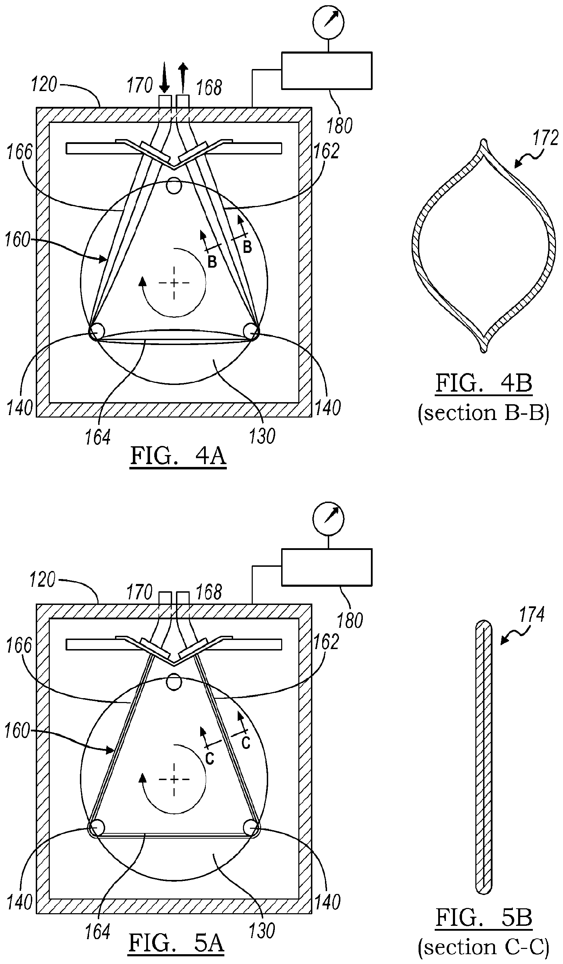

The fluid conduit 160 of the fluid pump functions to carry fluid flow through the fluid pump. As shown in FIGURES 4A and 5A, the fluid conduit 160 is preferably wrapped under tension around at least one of the plurality of rollers 140 (and, more preferably, around most or all of the rollers 140). The fluid conduit 160 preferably includes an inlet region 162, a body region 164, and an outlet region 166. The fluid conduit 160 preferably facilitates flow from the inlet region 162 to the outlet region 166. When the fluid pump is in operation, fluid flows into the inlet region 162 of the fluid conduit from the patient or a venous reservoir (which preferably includes blood ultimately from the blood circulation of the patient, but may alternatively be from a blood transfusion or any suitable source). As the plurality of rollers 140 advance across the fluid conduit, isolated fluid is captured in a region of the fluid conduit between a pair of adjacent rollers, expanding the fluid conduit. As rotation of the frame 130 causes the plurality of rollers 140 to rotate and advance further along the fluid conduit, the isolated fluid is shuttled from the inlet region 162 to the body region 164, and further into and out of the outlet region 166.

The fluid conduit 160 preferably operates in at least two modes: a filled mode 172 and a collapsed mode 174. As shown in FIGURE 4B, in the filled mode 172, the cross section of at least a portion of the fluid conduit 160 is expanded by the fluid, and rotation of the frame 130 causes the plurality of rollers 140 to compress the fluid conduit 160 at subsequent intervals, thereby driving the fluid to the outlet region 166 with a peristaltic movement. The fluid conduit 160 is in the filled mode 172 when fluid is supplied to the inlet region 162 at a pressure above the pressure outside the fluid conduit (e.g., above the vacuum level formed in the housing cartridge 120). When the fluid conduit 160 is in the filled mode 172, the fluid pressure within the fluid conduit 160 may be any pressure within a range of pressures, and flow rate preferably at least partially varies with the fluid pressure. At a characteristic fluid pressure, the fluid preferably slips past the rollers such that the fluid pressure does not further increase. This lack of increase in fluid pressure effectively limits the pumping power of the fluid pump, which may lessen danger to the patient or other fluid devices that lie downstream of the fluid pump. The lack of increase also lowers shear forces on the fluid conduit and decreases the likelihood of rupture of the fluid conduit. As shown in FIGURE 5B, in the collapsed mode 174, the cross section of at least a portion of the fluid conduit 160, such as the inlet region 162, is closed and prevents fluid flow towards the outlet region 166. The fluid conduit 160 is preferably in the collapsed mode 174 when the pressure inside the fluid conduit is equal to the pressure outside the fluid conduit (e.g., equal to the pressure level formed in the housing cartridge 120). When the fluid conduit 160 is in the collapsed mode 174, the fluid pump cannot generate suction greater (cannot generate more negative pressure) than the pressure level formed in the housing cartridge 120. Therefore, the pressure level in the housing cartridge 120 preferably sets the limit of suction that the fluid pump provides, and preferably is set to a level that avoids cavitation and hemolysis of the patient and/or damage to downstream components of the fluid pump or other equipment. The presence of the vacuum assists drainage of the fluid conduit, and also preferably enables operation of the fluid pump in any orientation of the fluid pump and/or patient. Furthermore, when the fluid conduit is in the collapsed mode 174, the plurality of rollers 140 do not pump any contents of the fluid conduit, but merely pass over the closed, flattened fluid conduit until the fluid conduit is in the filled mode 172. The transition between the collapsed mode 174 and the filled mode 172 occurs when pressure of the fluid supplied to the inlet region 162 is above the vacuum level in the housing cartridge 120. In other words, the blood volume supplied to the inlet region 162 volume must be above a certain volume ("prime volume") for the fluid conduit to transition to the filled mode 172. Conversely, the transition between the filled mode 172 to the collapsed mode 174 occurs when blood flow from the patient or venous reservoir into the fill region of the fluid conduit 160 slows. As the pressure of the fluid supplied to the fill region decreases, the fluid conduit 160 begins to collapse, as blood flow decreases in proportion to the decreased filling of the fill region. The fluid conduit may also operate in an occluded mode when the pressure within the fluid conduit is greater than the pressure outside the fluid conduit, in which the fluid conduit is at least partially open at the point of contact with a roller, such that fluid flow in the pump is either completely prevented, partially restricted, or reversed. The flow rate of the fluid pump is preferably dependent on revolutions per minute (RPM) of the frame 130, and on fluid filling pressure in the fill region of the fluid conduit 160.

The fluid conduit 160 is preferably made of medical-grade polyurethane, and more preferably from a profile extrusion of thermoplastic polyurethane for durability and greater strength and toughness than other thermoset materials. However, the fluid conduit may be made of any suitable material. The extrusion is preferably shaped in a secondary heat biasing process that optimizes the filling characteristics of the pump. In this secondary process, the profile of the fluid conduit 160 is preferably formed to be as flat as possible, while maintaining two characteristics: (1) sharp interior corners that minimize wear and fatigue by allowing the fluid conduit 160 to completely flatten at the roller locations without inducing high cyclical bending stresses along the edges of the fluid conduit; and (2) maintaining additional material on the edges that increases durability of the fluid conduit 160. The fluid conduit 160 may be further treated to include an internal coating of heparin, an anticoagulant, to reduce the tendency of blood clots as blood passes through the fluid pump. The extrusion is preferably cut to length and wrapped around the plurality of rollers 140 in factory assembly of the housing cartridge 120. Fixtures are preferably used to obtain a consistent length of fluid conduit 160, and to ensure that uniform tension is applied as the fluid conduit is wrapped around the plurality of rollers 140. A polyurethane inlet tube 168 is preferably radiofrequency (RF) welded to the inlet region 162 of the fluid conduit and attached to a mounting fitting that is ultraviolet (UV) light bonded to the housing cartridge 120. Similarly, a polyurethane outlet tube 170 is preferably RF welded to the outlet region 166 of the fluid conduit and attached to a second mounting fitting that is UV bonded to the housing cartridge 120. As shown in FIGURES 3 and 4, the inlet and outlet tubes are preferably mounted adjacent to one another on the housing cartridge 120. In some preferred embodiments, the fluid conduit 160 may have the geometry and construction of that described in U.S. Patent No. 5,486,099 entitled "Peristaltic pump with occlusive inlet" or in U.S. Patent Application No. 12/095,733 entitled "Pulsatile rotary ventricular pump.

In one very specific example of the fluid pump intended for a pediatric patient weighing less than 25 kilograms, the fluid pump provides a blood flow rate of 0-2.5 liters/minute. The pump motor is approximately 13.5cm (5.3 inches) in diameter, the frame is approximately 8.3cm (3.25 inches) inches in diameter, and the combined depth of the housing cartridge and pump motor is preferably approximately 7.9cm (3.1 inches). The frame includes three rollers distributed at the vertices of an equilateral triangle. The fluid conduit has a priming volume of less than 25 milliliters, and has a 9.5mm (3/8") diameter inlet tube 168 and a 6.3mm (1/4") diameter outlet tube welded to the fluid conduit.

The fluid pump may further include a controller that allows an operator to interact with, monitor, and control the fluid pump. The controller preferably includes a graphical user interface, a touch screen or buttons, an ultrasonic flow meter, a bubble detector, pressure sensors, a vacuum controller, and any suitable hardware. The controller preferably further includes an optical sensor located at the bottom end of the housing cartridge, which may be used to detect an obstruction such as pooling fluid levels in the bottom of the housing cartridge, which suggests a leakage in the fluid conduit. The controller preferably includes an audio and/or visual device to alert the user of the possible leak. The controller preferably also allows the user to define limits for triggering an alarm regarding various crucial characteristics of the fluid pump, such as flow rate and pressure.

### 2. Method of supplying a plurality of housing cartridges

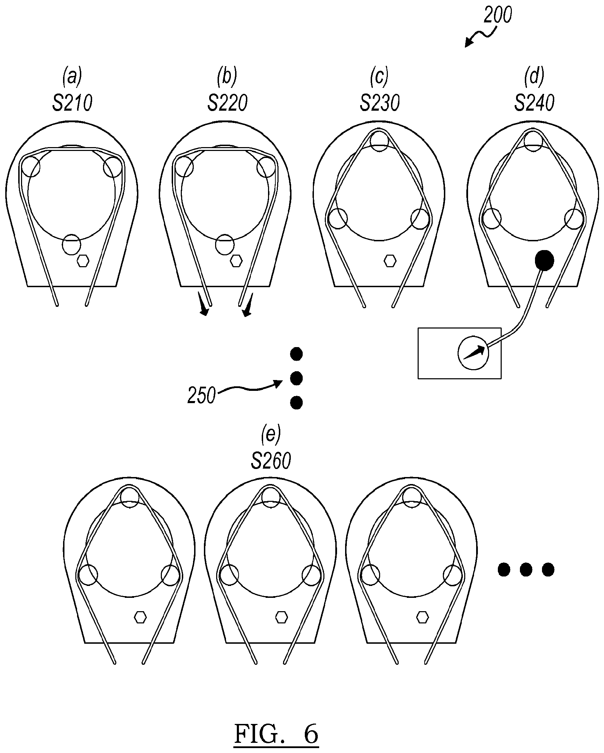

As shown in FIGURE 6, the method 200 of supplying a plurality of housing cartridges preferably includes the steps of: manufacturing a sealed housing cartridge S210 that includes manufacturing a rotating frame, a plurality of rollers arranged on the frame, and a fluid conduit wrapped around the plurality of rollers; tensioning the fluid conduit to a desired amount of tension S220; positioning the plurality of rollers to minimize the path length of the conduit around the rollers, thereby relieving at least a portion of tension in the fluid conduit S230. The method may optionally further include setting the internal pressure of the housing cartridge to a desired pressure level S240. The method 200 preferably further includes step S250, which includes repeating steps S210-S240 for supplying a plurality of housing cartridges, and step S260, which includes distributing the plurality of housing cartridges. The housing cartridge is preferably interchangeable with other housing cartridges in a fluid pump, and adapted to removably couple to a pump motor in the fluid pump.

Step S210, which includes manufacturing a sealed housing cartridge, preferably includes the manufacturing steps described above, to form a rotating frame, a plurality of rollers arranged on the frame, and a fluid conduit wrapped around the plurality of rollers. The rotating frame preferably has a portion of a drive coupling that mates with another portion of the drive coupling connected to the pump motor.

Step S220, which includes tensioning the fluid conduit to a desired amount of tension, functions to tighten the fluid conduit around the rollers to a suitable tightness setting to limit the maximum amount of fluid pressure that, when the housing cartridge is coupled to the pump motor, limits the maximum amount of fluid pressure that can be generated in the fluid conduit. S220 preferably includes wrapping the fluid conduit around a fixture that ensures that uniform tension is applied as the fluid conduit is wrapped around the plurality of rollers.

Step S230, which includes positioning the plurality of rollers, functions to relieve tension in the fluid conduit to reduce creep that a fluid conduit continuously loaded in tension may experience over a long period of time such as during storage and/or shipping. Step S230 preferably positions the plurality of rollers such that the path length of the conduit around the rollers is minimized. Step S230 preferably includes rotating the frame, which preferably rotates the orientation of the plurality of rollers such that the path length of the fluid conduit around the rollers is minimized. For example, as shown in FIGURE 6C, in a housing cartridge having a frame with three rollers equally distributed around the circumference of the frame, the frame is preferably rotated such that all three rollers are in contact with the fluid conduit. In some embodiments, the frame may have many rollers, such that there is no rotational orientation of the frame in which all the rollers are in contact with the fluid conduit. In these embodiments, it may be sufficient that the rollers are positioned to allow as many rollers as possible to contact the fluid conduit. Step S230 may further include locking the positions of the rollers, such as with a latch or other suitable mechanism that sets the rotational position of the frame.

Step S240, which includes setting the internal pressure of the housing cartridge to a desired pressure level S240, functions to limit the amount of suction that the fluid pump provides, when the housing cartridge is coupled to the pump motor. Through a pressure control port on the housing cartridge, the internal pressure is preferably set to a level that avoids cavitation and hemolysis of the patient and/or damage to downstream components of the fluid pump or other equipment. The internal pressure of the housing cartridge is preferably set to a vacuum, but may alternatively be set to a positive pressure. After setting the internal pressure of the housing cartridge, the pressure control port may be sealed or eliminated.

Step S250, which includes repeating steps S210-S240 for a plurality of housing cartridges preferably increases consistency between housing cartridges that are distributed, such that a fluid pump that is compatible with the distributed housing cartridges performs more reliably and consistently between interchanged housing cartridges.

Step 260, which includes distributing the plurality of housing cartridges, functions to provide a series of cartridges to a customer, such as a hospital. The series of cartridges are preferably provided in a case, such as a case of 24 cartridges, but may be provided in any suitable number or as a single cartridge on an as-needed basis.

As a person skilled in the art will recognize from the previous detailed description and from the figures and claims, modifications and changes can be made to the preferred embodiments of the invention without departing from the scope of this invention defined in the following claims.

## Claims

1. A housing cartridge (120) for a modular fluid pump system that includes a pump motor (110) providing a motor force, the housing cartridge comprising:
a frame (130) enclosed in the housing cartridge;
a plurality of rollers (140) arranged on the frame;
a second mating portion (154) of drive coupling means (150) connected to the frame, wherein the second mating portion (154) is able to mate in use with a first mating portion (152) of drive coupling means connected to the pump motor (110), such that the drive coupling means transmits the motor force to the frame thereby inducing rotation of the frame; and
a fluid conduit (160), wrapped under tension around at least one of the plurality of rollers, that facilitates fluid flow from an inlet region of the fluid conduit to an outlet region of the fluid conduit, wherein rotation of the frame causes the plurality of rollers to compress the fluid conduit at subsequent intervals, thereby driving fluid to the outlet region with a peristaltic movement;
wherein the housing cartridge is interchangeable with a replacement housing cartridge in the modular fluid pump system;
**characterized in that**:
the housing cartridge is airtight sealed and includes a pressure control port (122) that facilitates adjustment of the pressure within the housing cartridge.

2. The housing cartridge of Claim 1, wherein the fluid conduit operates in the following modes:
a filled mode, wherein the cross section of at least a portion of the fluid conduit is expanded by the fluid and allows fluid flow toward the outlet region during rotation of the frame; and
a collapsed mode, wherein the cross section of at least the inlet region is closed and prevents fluid flow toward the outlet region during rotation of the frame.

3. The housing cartridge of Claim 2, wherein fluid flow in the fluid conduit decreases in proportion to decreased filling pressure at the inlet region.

4. The housing cartridge of Claim 2, wherein the fluid conduit further operates in an occluded mode, wherein the pressure within the fluid conduit is greater than the pressure in the housing cartridge, wherein the fluid conduit is at least partially open at the point of contact with at least one of the plurality of rollers.

5. A modular fluid pump (100) comprising:
a pump motor (110) that provides a motor force; and
a housing cartridge (120) according to any one of the preceding claims, removably coupled to the pump motor, wherein the housing cartridge is interchangeable with a replacement housing cartridge;
said drive coupling means transmitting in use the motor force from the pump motor to the frame thereby inducing rotation of the frame.

6. The modular fluid pump of Claim 5, wherein the drive coupling means includes a magnetic coupler (156).

7. The modular fluid pump of Claim 6, wherein the magnetic coupler is a magnetic disc coupler including a first disc (152) and a second disc (154) magnetically attracted to the first disc, wherein the first disc is connected to the pump motor and the second disc is connected to the frame.

8. The modular fluid pump of Claim 6, wherein the magnetic coupler is a magnetic coaxial coupler including a first portion (152) and a second portion (154) magnetically attracted to the first portion, wherein the first portion is connected to the pump motor and the second portion is connected to the frame.

9. The modular fluid pump of Claim 7 or 8, wherein at least one of the first and second portions of the magnetic coaxial coupler is enclosed in an airtight seal.

10. The modular fluid pump of Claim 5, wherein the pump motor is coupled to a motor housing (112) having a mating surface (114) that mates with the housing cartridge.

11. The modular fluid pump of Claim 5, wherein the drive coupling means includes a torque-limiting mechanism (151).

12. A method of supplying a housing cartridge, comprising:
manufacturing a sealed housing cartridge including: a rotating frame, a plurality of rollers arranged on the frame, and a fluid conduit wrapped around the plurality of rollers, wherein the housing cartridge is airtight sealed and includes a pressure control port that facilitates adjustment of the pressure within the housing cartridge;
tensioning the fluid conduit to a desired amount of tension; and
positioning the plurality of rollers to minimize the path length of the fluid conduit around the rollers, thereby removing at least a portion of tension in the fluid conduit.

13. The method of Claim 12, wherein the step of positioning the plurality of rollers includes rotating the frame.

14. The method of Claim 12, further comprising the step of forming a vacuum within the housing cartridge.

## Patentansprüche

1. Gehäusekartusche (120) für ein modulares Fluidpumpensystem, das einen Pumpenmotor (110) umfasst, der eine Motorkraft bereitstellt, wobei die Gehäusekartusche Folgendes umfasst:
einen Rahmen (130), der in der Gehäusekartusche eingeschlossen ist;
eine Vielzahl von Rollen (140), die an dem Rahmen angeordnet sind;
einen zweiten Passabschnitt (154) von Antriebskopplungsmitteln (150), die mit dem Rahmen verbunden sind, wobei der zweite Passabschnitt (154) in der Lage ist, im Gebrauch mit einem ersten Passabschnitt (152) von Antriebskopplungsmitteln zusammenzupassen, die mit dem Pumpenmotor (110) verbunden sind, so dass das Antriebskopplungsmittel die Motorkraft auf den Rahmen überträgt, wodurch eine Drehung des Rahmens induziert wird; und
eine Fluidleitung (160), die unter Spannung um mindestens eine der Vielzahl von Rollen gewickelt ist und die Fluidströmung von einem Einlassbereich der Fluidleitung zu einem Auslassbereich der Fluidleitung ermöglicht, wobei die Drehung des Rahmens bewirkt, dass die Vielzahl von Rollen die Fluidleitung in nachfolgenden Intervallen zusammendrücken, wodurch Flüssigkeit mit einer peristaltischen Bewegung in den Auslassbereich getrieben wird;
wobei die Gehäusekartusche mit einer Ersatzgehäusekartusche in dem modularen Fluidpumpensystem austauschbar ist;
**dadurch gekennzeichnet, dass**:
die Gehäusekartusche luftdicht verschlossen ist und eine Druckregelöffnung (122) umfasst, die die Einstellung des Drucks in der Gehäusekartusche ermöglicht.

2. Gehäusekartusche nach Anspruch 1, wobei die Fluidleitung in den folgenden Betriebsarten arbeitet:
einem gefüllten Modus, bei dem der Querschnitt von mindestens einem Teil der Fluidleitung durch das Fluid erweitert wird und einen Fluidfluss in Richtung des Auslassbereichs während der Drehung des Rahmens ermöglicht; und
einem kollabierten Modus, bei dem der Querschnitt mindestens des Einlassbereichs geschlossen ist und eine Fluidströmung in Richtung des Auslassbereichs während der Drehung des Rahmens verhindert.

3. Gehäusekartusche nach Anspruch 2, wobei die Fluidströmung in der Fluidleitung proportional zu einem verringerten Befüllungsdruck in dem Einlassbereich abnimmt.

4. Gehäusekartusche nach Anspruch 2, wobei die Fluidleitung ferner in einem verschlossenen Modus arbeitet, wobei der Druck in der Fluidleitung größer ist als der Druck in der Gehäusekartusche, wobei die Fluidleitung an der Kontaktstelle mit mindestens einer der Vielzahl von Rollen mindestens teilweise offen ist.

5. Modulare Fluidpumpe (100), die Folgendes umfasst:
einen Pumpenmotor (110), der eine Motorkraft bereitstellt;
eine Gehäusekartusche (120) nach einem der vorhergehenden Ansprüche, die abnehmbar mit dem Pumpenmotor verbunden ist, wobei die Gehäusekartusche gegen eine Ersatzgehäusekartusche austauschbar ist;
wobei das Antriebskopplungsmittel im Gebrauch die Motorkraft vom Pumpenmotor auf den Rahmen überträgt, wodurch eine Drehung des Rahmens induziert wird.

6. Modulare Fluidpumpe nach Anspruch 5, wobei das Antriebskopplungsmittel einen Magnetkoppler (156) umfasst.

7. Modulare Fluidpumpe nach Anspruch 6, wobei der Magnetkoppler ein Magnetplattenkoppler ist, der eine erste Scheibe (152) und eine zweite Scheibe (154) umfasst, die magnetisch zu der ersten Scheibe angezogen wird, wobei die erste Scheibe mit dem Pumpenmotor verbunden ist und die zweite Scheibe mit dem Rahmen verbunden ist.

8. Modulare Fluidpumpe nach Anspruch 6, wobei der Magnetkoppler ein magnetischer Koaxialkoppler ist, der einen ersten Abschnitt (152) und einen zweiten Abschnitt (154) umfasst, der magnetisch zu dem ersten Abschnitt angezogen wird, wobei der erste Abschnitt mit dem Pumpenmotor verbunden ist und der zweite Abschnitt mit dem Rahmen verbunden ist.

9. Modulare Fluidpumpe nach Anspruch 7 oder 8, wobei mindestens einer der ersten und zweiten Abschnitte des magnetischen Koaxialkopplers in einer luftdichten Dichtung eingeschlossen ist.

10. Modulare Fluidpumpe nach Anspruch 5, wobei der Pumpenmotor mit einem Motorgehäuse (112) gekoppelt ist, das eine Passfläche (114) aufweist, die mit der Gehäusekartusche zusammenpasst.

11. Modulare Fluidpumpe nach Anspruch 5, wobei das Antriebskopplungsmittel einen Drehmomentbegrenzungsmechanismus (151) umfasst.

12. Verfahren zum Zuführen einer Gehäusekartusche, das Folgendes umfasst:
Herstellen einer versiegelten Gehäusekartusche, die Folgendes umfasst: einen Drehrahmen, eine Vielzahl von Rollen, die auf dem Rahmen angeordnet sind, und eine Fluidleitung, die um die Vielzahl von Rollen gewickelt ist, wobei die Gehäusekartusche luftdicht verschlossen ist und eine Druckregelöffnung umfasst, die die Einstellung des Drucks in der Gehäusekartusche ermöglicht;
Spannen der Fluidleitung auf einen gewünschten Spannungsbetrag; und
Positionieren der Vielzahl von Rollen, um die Weglänge der Fluidleitung um die Rollen herum zu minimieren, wodurch zumindest ein Teil der Spannung in der Fluidleitung entfernt wird.

13. Verfahren nach Anspruch 12, wobei der Schritt des Positionierens der Vielzahl von Rollen das Drehen des Rahmens umfasst.

14. Verfahren nach Anspruch 12, das ferner den Schritt des Bildens eines Vakuums in der Gehäusekartusche umfasst.

## Revendications

1. Cartouche de logement (120) pour système de pompe modulaire à fluide qui comporte un moteur de pompe (110) produisant une force moteur, la cartouche de logement comprenant :
un cadre (130) enfermé dans la cartouche de logement ;
une pluralité de rouleaux (140) agencée sur le cadre ;
une seconde partie d'accouplement (154) d'un moyen de couplage d'entraînement (150) connectée au cadre, la seconde partie d'accouplement (154) étant en mesure de s'accoupler en service avec une première partie d'accouplement (152) du moyen de couplage d'entraînement connecté au moteur de pompe (110), de telle sorte que le moyen de couplage d'entraînement transmette la force moteur au cadre induisant ainsi une rotation du cadre ; et
un conduit de fluide (160), enroulé en tension autour d'au moins un de la pluralité de rouleaux, qui facilite l'écoulement de fluide depuis une région d'entrée du conduit de fluide jusqu'à une région de sortie du conduit de fluide, dans lequel la rotation du cadre amène la pluralité de rouleaux à comprimer le conduit de fluide à intervalles subséquents, entraînant ainsi le fluide jusqu'à la région de sortie avec un mouvement péristaltique ;
dans lequel la cartouche de logement est interchangeable avec une cartouche de logement de remplacement dans le système de pompe modulaire à fluide ;
**caractérisé en ce que** :
la cartouche de logement est étanche à l'air et comporte un orifice de commande de pression (122) qui facilite l'ajustement de la pression à l'intérieur de la cartouche de logement.

2. Cartouche de logement selon la revendication 1, dans laquelle le conduit de fluide fonctionne dans les modes suivants :
un mode rempli, dans lequel la section transversale d'au moins une partie du conduit de fluide est élargie par le fluide et permet un écoulement de fluide vers la région de sortie durant la rotation du cadre ; et
un mode comprimé, dans laquelle la section transversale d'au moins la région d'entrée est fermée et empêche l'écoulement de fluide vers la région de sortie durant la rotation du cadre.

3. Cartouche de logement selon la revendication 2, dans laquelle l'écoulement de fluide dans le conduit de fluide diminue proportionnellement à la réduction de la pression de remplissage au niveau de la région d'entrée.

4. Cartouche de logement selon la revendication 2, dans laquelle le conduit de fluide fonctionne en outre dans un mode d'occlusion, dans lequel la pression à l'intérieur du conduit de fluide est supérieure à la pression dans la cartouche de logement, dans laquelle le conduit de fluide est au moins partiellement ouvert au point de contact avec au moins un de la pluralité de rouleaux.

5. Pompe modulaire à fluide (100) comprenant :
un moteur de pompe (110) qui produit une force moteur ;
une cartouche de logement (120) selon l'une quelconque des revendications précédentes, couplée de manière amovible au moteur de pompe, la cartouche de logement étant interchangeable avec une cartouche de logement de remplacement ;
ledit moyen de couplage d'entraînement transmettant en service la force moteur du moteur de pompe au cadre induisant ainsi une rotation du cadre.

6. Pompe modulaire à fluide selon la revendication 5, dans laquelle le moyen de couplage d'entraînement comporte un coupleur magnétique (156).

7. Pompe modulaire à fluide selon la revendication 6, dans laquelle le coupleur magnétique est un coupleur à disques magnétiques comportant un premier disque (152) et un second disque (154) attiré magnétiquement vers le premier disque, dans laquelle le premier disque est connecté au moteur de pompe et le second disque est connecté au cadre.

8. Pompe modulaire à fluide selon la revendication 6, dans laquelle le coupleur magnétique est un coupleur coaxial magnétique comportant une première partie (152) et une seconde partie (154) attirée magnétiquement vers la première partie, dans laquelle la première partie est connectée au moteur de pompe et la seconde partie est connectée au cadre.

9. Pompe modulaire à fluide selon la revendication 7 ou 8, dans laquelle au moins une des première et seconde parties du coupleur coaxial magnétique est enfermée d'une manière étanche à l'air.

10. Pompe modulaire à fluide selon la revendication 5, dans laquelle le moteur de pompe est couplé à un carter de moteur (112) présentant une surface d'accouplement (114) qui s'accouple avec la cartouche de logement.

11. Pompe modulaire à fluide selon la revendication 5, dans laquelle le moyen de couplage d'entraînement comporte un mécanisme de limitation de couple (151).

12. Procédé de fourniture d'une cartouche de logement, comprenant :
la fabrication d'une cartouche de logement scellée comportant : un cadre rotatif, une pluralité de rouleaux agencée sur le cadre, et un conduit de fluide enveloppé autour de la pluralité de rouleaux, dans lequel la cartouche de logement est étanche à l'air et comporte un orifice de commande de pression qui facilite l'ajustement de la pression à l'intérieur de la cartouche de logement ;
la mise en tension du conduit de fluide jusqu'à une quantité de tension souhaitée ; et
le positionnement de la pluralité de rouleaux pour minimiser la longueur de trajet du conduit de fluide autour des rouleaux, éliminant ainsi au moins une partie de la tension dans le conduit de fluide.

13. Procédé selon la revendication 12, dans laquelle l'étape de positionnement de la pluralité de rouleaux comporte la rotation du cadre.

14. Procédé selon la revendication 12, comprenant en outre l'étape de formation d'un vide à l'intérieur de la cartouche de logement.
